Europäisches Patentamt

**European Patent Office**

Office européen des brevets

(19)

(11) Veröffentlichungsnummer: **0 057 390**
A2

(12) # EUROPÄISCHE PATENTANMELDUNG

(21) Anmeldenummer: **82100435.5**

(22) Anmeldetag: **22.01.82**

(51) Int. Cl.³: **G 01 N 33/18,** G 01 N 31/00

(30) Priorität: **29.01.81 DE 3102903**

(43) Veröffentlichungstag der Anmeldung: **11.08.82**
**Patentblatt 82/32**

(84) Benannte Vertragsstaaten: **AT BE CH DE FR GB IT LI NL SE**

(71) Anmelder: **HOECHST AKTIENGESELLSCHAFT,
Postfach 80 03 20, D-6230 Frankfurt/Main 80 (DE)**

(72) Erfinder: **Melzer, Werner, Dr., Drosselweg 2,
D-6237 Liederbach (DE)**
Erfinder: **Jaenicke, Dieter, Am Helgenstock 10,
D-6238 Hofheim am Taunus (DE)**

(54) Verfahren zum Austreiben von anorganisch gebundenem Kohlenstoff aus Wasser.

(57) Für die Bestimmung des organisch gebundenen Kohlenstoffs (TOC) in Wasser muß, um Fehlmessungen zu vermeiden, der anorganisch gebundene Kohlenstoff vor einer TOC-Bestimmung quantitativ entfernt werden. Hierfür wird das Wasser mit einer Mineralsäure angesäuert und das gebildete $CO_2$ mit Gas ausgetrieben. Das mit $CO_2$ beladene Gas wird anschließend über einen $CO_2$-Filter geleitet und vollständig dem angesäuerten Wasser wieder zugeführt. Dabei darf dem Gaskreislauf $CO_2$-freies Gas weder zugeführt noch entnommen werden.

## Verfahren zum Austreiben von anorganisch gebundenem Kohlenstoff aus Wasser

Die Erfindung betrifft ein Verfahren zum Austreiben von anorganisch gebundenem Kohlenstoff aus Wasser durch Ansäuern des Wassers mit einer Mineralsäure und Austreiben des gebildeten $CO_2$ mit Gas, wobei das mit $CO_2$ beladene Gas über ein $CO_2$-Filter geleitet und teilweise dem angesäuerten Wasser wieder zugeführt wird.

Bei der Bestimmung des organischen Kohlenstoffs (TOC) in Wasser wird anorganisch gebundener Kohlenstoff, wie er in allen natürlichen Wässern in Form von $CO_2$, $HCO_3^-$ und $CO_3^{--}$ vorliegt, grundsätzlich mit erfaßt. Er muß entweder vorher entfernt oder aber gesondert bestimmt und vom Meßergebnis abgezogen werden.

Nach G. Axt, Haus der Technik - Vortragsveröffentlichungen, Heft 231 (1970) Seite 21 bis 28 ist ein Verfahren zum Entfernen von anorganisch gebundenem Kohlenstoff aus Wasser der genannten Art bekannt. Nachteilig bei diesem Verfahren ist, daß die Ansäuerung nur in dem relativ kleinen Abwasserstrom geschehen kann, der auch dem Verbrennungsofen des nachgeschalteten TOC-Analysators zugeführt wird. Wird nur ein Teilstrom der angesäuerten Probe zur TOC-Messung verwendet, gehen auch flüchtige Kohlenwasserstoffe verloren, die Messung wird verfälscht.

Hier will die Erfindung Abhilfe schaffen. Die Erfindung löst die Aufgabe dadurch, daß dem Gaskreislauf $CO_2$-freies Gas weder zugeführt noch entnommen wird.

Zur Austragung von $CO_2$ aus der angesäuerten Probe wird ein geschlossener von der TOC-Analyse völlig getrennter Gaskreislauf verwendet. Auf diese Weise läßt sich ein relativ großer Probestrom von anorganischen Kohlenstoff-

anteilen fehlerfrei befreien und ein Teilstrom ohne Verluste an flüchtigen Kohlenwasserstoffen ausmessen.

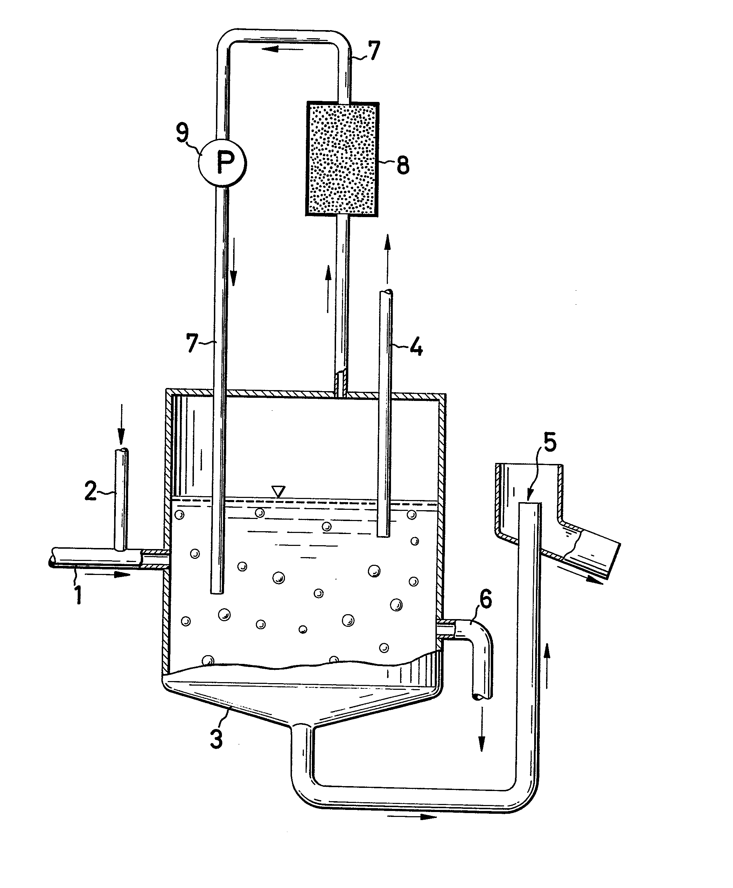

Das erfindungsgemäße Verfahren wird im Folgenden anhand der Figur näher erläutert.

Für die Messung bestimmtes Wasser, dem über Leitung 2 Säure zugeführt wird, wird kontinuierlich oder diskontinuierlich über Leitung 1 in Gefäß 3 geleitet, in dem ein bestimmtes Flüssigkeitsniveau mittels Überlauf 5 gehalten wird. Gas, zum Beispiel Luft, durchperlt das im Gefäß 3 befindliche Wasser und trägt dabei $CO_2$ und gegebenenfalls flüchtige organische Kohlenwasserstoffverbindungen aus. In einem $CO_2$-Filter 8 wird die Luft von $CO_2$ gereinigt und über Leitung 7 mittels Pumpe 9, zum Beispiel einer Membranpumpe, dem Wasser in Gefäß 3 wieder zugeführt. Nach kurzer Betriebszeit werden keine flüchtigen organischen Kohlenstoffverbindungen mehr ausgetragen, da sich ein Sättigungsgleichgewicht zwischen Luft und Wasser eingestellt hat. Das für die Analyse benötigte Wasser wird nun über Leitung 6 Gefäß 3 entnommen. Leitung 4 sorgt für die Entlüftung von Gefäß 3.

0057390

HOE 81/F 024

Patentanspruch

Verfahren zum Austreiben von anorganisch gebundenem Kohlenstoff aus Wasser durch Ansäuern des Wassers mit einer
Mineralsäure und Austreiben des gebildeten $CO_2$ mit Gas,
wobei das mit $CO_2$ beladene Gas über ein $CO_2$-Filter geleitet
und teilweise dem angesäuerten Wasser wieder zugeführt
wird, dadurch gekennzeichnet, daß dem Gaskreislauf
$CO_2$-freies Gas weder zugeführt noch entnommen wird.